# EUROPEAN PATENT APPLICATION

(11) **EP 2 737 804 A1**
(43) Date of publication of application: **04.06.2014**
(21) Application number: 13157719.9
(22) Date of filing: 05.03.2013
(51) Int. Cl.: A23C 9/152, A23D 7/01, A23J 7/00, A61K 31/685, A23L 1/03

(54) **Processes for the preparation of phospholipid-enriched dairy products as neutraceuticals for the formulation of functional foods, and neutraceutical and/or pharmaceutical compositions thereof**

(30) Priority: 02.12.2012 IL 22337312
(71) Applicant: Lipogen Ltd., 34372 Haifa (IL)
(72) Inventor: Rutenberg, David, 34372 Haifa (IL); Perry, Ilan, 30992 Moshav Bat Shlomo (IL)
(74) Representative: Modiano, Micaela Nadia

(57) **Abstract**

The present invention discloses processes for the preparation of phospholipid-enriched dairy products as nutraceuticals for the formulation of functional foods, and nutraceutical and/or pharmaceutical compositions thereof, the processes include the steps of: combining a non-dairy-based PL-containing material with an oil component and water to form a paste; removing an excess amount of the water from the paste to form a PL-oil solution; and mixing the PL-oil solution with a dairy component, thereby obtaining a PL-enriched dairy product. Preferably, the PL-containing material includes at least one material selected from the group consisting of: a vegetal-derived lecithin, a non-vegetal-derived lecithin, a de-oiled lecithin, a native lecithin-oil solution, and an enzymatically-processed lecithin. Preferably, the PL-oil solution has a weight-to-weight (w-w) concentration of at least about 0.01% of a residual amount of the water to the PL-containing material.

## Description

### CROSS REFERENCE TO RELATED APPLICATIONS

This patent application claims priority to Israel Patent Application No. 223373 filed December 2, 2012, which is hereby incorporated by reference in its entirety.

### FIELD AND BACKGROUND OF THE INVENTION

The present invention relates to processes for the preparation of phospholipid-enriched dairy products as nutraceuticals for the formulation of functional foods, and to nutraceutical and/or pharmaceutical compositions thereof.

Phospholipids (PLs) are ubiquitous biological substances, making up the membrane material in most cells in both plants and animals. PLs have been used extensively in pharmaceutical compositions, nutritional compounds, and functional foods. As an example, the importance of phosphatidylserine (PS) as a functional ingredient is supported by the US FDA's qualified health claims in which the usage of phosphatidylserine was related to the reduction of cognitive dysfunction and dementia in the elderly.

PLs can be used as a nutraceutical (including a medication, a medical food, a functional food, and a dietary supplement) in various food formulations, drinks, tablets, and bars containing concentrated, nutritional and/or dietary ingredients. The addition of PLs to dairy formulations is exacerbated by undesirable attributes of a finished product such as unpleasant taste, non-homogenous consistency, unattractive appearance, and/or poor fractionation stability, among others.

US Patent No. 8,231,922 by Burling et al. (hereinafter referred to as Burling '922) recites a bovine-milk-derived, phosphatidylserine source of natural composition having excellent dispersibility and organoleptic as well as physical stability using PS-enriched milk fractions. As disclosed in Burling '922, natural milk is a poor source of PS, with only ∼27 mg PS/liter in whole milk and ∼10 mg PS/liter in skim milk. Buttermilk provides a better source of PS, with cream-churned buttermilk containing ∼130 mg PS/liter, while butter-oil-derived buttermilk contains ∼250 mg PS/liter. In a society concerned with reducing dietary fat intake, the loss of naturally-occurring PS during the skimming process of removing fat from milk leaves the nutritional benefit substantially depleted. The skimming process is thought to deplete the naturally-occurring PLs in the milk.

Burling '922 further discloses test results for a milk-added, buttermilk-derived PS (2.0% PS) being physically-, chemically-, and organoleptically-stable, with no precipitation. Milk-added, soy-derived PS (21% and 62% from Enzymotec, Israel) was found to be physically unstable, with 70% of the PS precipitating and settling after one week. Moreover, the milk-added, soy-derived PS was found to be organoleptically unstable, acquiring an unpleasant off-taste typical for soy that made the milk almost undrinkable. All comparative formulations were standardized to provide 100 mg of PS for a 200 ml serving of a skim-milk based drink.

It would be desirable to have processes for the preparation of PL-enriched dairy products as nutraceuticals for the formulation of functional foods, and nutraceutical and/or pharmaceutical compositions thereof, including non-dairy-derived PS. Such processes and compositions would, *inter alia,* overcome the limitations mentioned above.

### SUMMARY OF THE INVENTION

It is the purpose of the present invention to provide processes for the preparation of PL-enriched dairy products as nutraceuticals (including medications, medical foods, functional foods, nutritional supplement, and dietary supplements) for the formulation of functional foods, and nutraceutical and/or pharmaceutical compositions thereof.

In the interest of clarity, the term "nutraceutical" is specifically defined for use herein to refer to any edible substance that is used in a medication, medical food, functional food, nutritional supplement, a pharmaceutical supplement, or dietary supplement, and provides medical and/or health benefits, including the prevention and treatment of disease.

Furthermore, it is noted that the term "exemplary" is used herein to refer to examples of embodiments and/or implementations, and is not meant to necessarily convey a more-desirable use-case. Similarly, the term "preferred" is used herein to refer to an example out of an assortment of contemplated embodiments and/or implementations, and is not meant to necessarily convey a more-desirable use-case. Therefore, it is understood from the above that "exemplary" and "preferred" may be applied herein to multiple embodiments and/or implementations.

Embodiments of the present invention provide processes for the preparation of PL-enriched dairy products as nutraceuticals for the formulation of functional foods, and nutraceutical and/or pharmaceutical compositions thereof. Such PL enrichment can serve as a nutraceutical ingredient for supplemental, dairy formulations. Such enriched dairy products include, but are not limited to, nutraceutical formulations of milk, milk chocolate, milk-ingredient supplemented products (e.g., enriched milk ingredients used in a powdered coffee or cocoa formulation), ice cream, dairy drinks, yoghurt, processed cheeses, cottage cheeses, dairy spreads, powdered dairy products, and nutritional dairy bars.

Therefore, according to the present invention, there is provided for the first time a process for the preparation of phospholipid-enriched (PL-enriched) dairy products as nutraceuticals for the formulation of functional foods, the process including the steps of: (a) combining a non-dairy-based PL-containing material with an oil component and water to form a paste; (b) removing an excess amount of the water from the paste to form a PL-oil solution; and (c) mixing the PL-oil solution with a dairy component, thereby obtaining a PL-enriched dairy product.

Preferably, the PL-enriched dairy product has the form of at least one nutraceutical type selected from the group consisting of: a medication, a medical food, a medical drink, a functional food, a functional drink, a nutritional supplement, a pharmaceutical supplement, and a dietary supplement.

Preferably, the PL-containing material includes at least one material selected from the group consisting of: a vegetal-derived lecithin, a non-vegetal-derived lecithin, a de-oiled lecithin, a native lecithin-oil solution, and an enzymatically-processed lecithin.

Preferably, the oil component includes at least one material selected from the group consisting of: a carrier oil and a native oil fraction from a lecithin production process.

Preferably, the step of combining is performed at a weight-to-weight (w-w) concentration of at least about 2% of the water to the PL-containing material.

Preferably, the step of removing is performed by at least one process selected from the group consisting of: a heating procedure, a vacuum-distillation procedure, and a solvent-based phase-separation procedure.

Preferably, the PL-oil solution has a weight-to-weight (w-w) concentration of at least about 0.01% of a residual amount of the water to the PL-containing material.

Preferably, the PL-oil solution has a weight-to-weight (w-w) concentration of about 10-80% of the PL-containing material to the oil component.

Preferably, the step of mixing includes at least one process selected from the group consisting of: a stirring procedure, a homogenizing procedure, and a pasteurizing procedure.

Preferably, the PL-enriched dairy product has a weight-to-weight (w-w) concentration of up to about 50% of the PL-oil solution to the dairy component.

Preferably, the PL-enriched dairy product is used in at least one product form selected from the group consisting of: a milk product, a chocolate product, a milk-ingredient supplemented product, an ice cream, a drink, a yoghurt, a processed cheese, a cottage cheese, a spread, a powdered dairy product, and a nutritional bar.

According to the present invention, there is provided for the first time a nutraceutical and/or pharmaceutical composition produced according to the process detailed above.

Preferably, the PL-enriched dairy product is in the form of at least one nutraceutical type selected from the group consisting of: a medication, a medical food, a medical drink, a functional food, a functional drink, a nutritional supplement, a pharmaceutical supplement, and a dietary supplement.

Preferably, the PL-enriched dairy product is used in at least one product form selected from the group consisting of: a milk product, a chocolate product, a milk-ingredient supplemented product, an ice cream, a drink, a yoghurt, a processed cheese, a cottage cheese, a spread, a powdered dairy product, and a nutritional bar.

These and further embodiments will be apparent from the detailed description and examples that follow.

### DESCRIPTION OF THE PREFERRED EMBODIMENTS

The present invention relates to processes for the preparation of PL-enriched dairy products as nutraceuticals for the formulation of functional foods, and to nutraceutical and/or pharmaceutical compositions thereof. The principles and operation for preparing such PL-enriched dairy products, according to the present invention, may be better understood with reference to the accompanying description. Exemplary embodiments of the present invention are detailed below in the following exemplary process.

### EXAMPLE 1:

A carrier oil (BERGABEST MCT Oil 60/40, Sternchemie GmbH & Co. KG) was combined with de-oiled lecithin (SternPur S P, Sternchemie GmbH & Co. KG) and 5% weight-to-weight (w-w) concentration of water to lecithin to form a uniform paste. The paste was then heated under vacuum to remove the excess water to yield a lecithin-oil solution having at least about 0.01% residual water. It is noted that a solvent-based phase-separation method can be used instead of (or in addition to) the vacuum distillation procedure. The w-w concentration of water to lecithin of at least about 2% was found to be amenable to the process. The w-w concentration of lecithin to oil used was 60%. A w-w concentration of lecithin to oil range of 10-80% was found to be amenable to the process with regard to solubility and stability. Any source of lecithin can be used including vegetal-derived lecithin (e.g., soybean lecithin, sunflower lecithin, and rapeseed lecithin) and non-vegetal-derived lecithin (e.g., egg yolk lecithin and fish lecithin).

The lecithin-oil solution was then combined with skim milk at a concentration of 10% w-w lecithin-oil solution to milk. A range of up to about 50% w-w concentration was found to be amenable to the process with regard to solubility and stability. The mixture was then stirred gently. Vigorous stirring at this stage can result in layer fractionation which produces cream. The PL-fortified milk was then homogenized. It is thought that the natural components found in milk act as emulsifiers in the process. The PL-fortified milk was finally pasteurized to protect against spoilage.

A representative PL profile for the PL-fortified milk included: phosphatidylserine (PS) at 0%, phosphatidic acid (PA) at 7%, phosphatidylcholine (PC) at 49%, phosphatidylinositol (PI) at 14%, and phosphatidylethanolamine (PE) at 30%. Percentages represent the weight fraction of the relevant PL component out of a total PL content based on PS, PA, PC, PI, and PE combined.

### EXAMPLE 2:

Lecithin with its native oil fraction associated with the production of lecithin can also be used. The native lecithin-oil solution (Lecisoy, Cargill, Inc.) used had a concentration of lecithin to oil of 50% w-w. The concentration can be adjusted by either removing a portion of the oil fraction, or by adding a carrier oil. A range of 10-80% w-w concentration was found to be amenable to the process with regard to solubility and stability. Any source of lecithin can be used including vegetal-derived lecithin and non-vegetal-derived lecithin.

The lecithin-oil solution was then combined with whole milk at a concentration of 5% w-w. A range of up to about 50% w-w concentration was found to be amenable to the process with regard to solubility and stability. The mixture was then stirred gently. Vigorous stirring at this stage can result in layer fractionation which produces cream. The PL-fortified milk was then homogenized. The PL-fortified milk was finally pasteurized to protect against spoilage.

A representative PL profile for the PL-fortified milk included: PS at 0%, PA at 5%, PC at 42%, PI at 20%, and PE at 33%. Percentages represent the weight fraction of the relevant PL component out of a total PL content based on PS, PA, PC, PI, and PE combined.

### EXAMPLE 3:

Enzymatically-processed lecithin can also be used, allowing the ratio of PL components (e.g., PS and PA) to be modified in order to obtain the desired type of nutraceutical product. Depending on the type of industrial PL material used, a minimum moisture content of at least about 0.01% w-w concentration of water to PL material may already be present in the commercially-available material. If the moisture content is below this threshold, then the procedure used in Example 1 can be followed. If the moisture content is above this threshold, then the procedure used in Example 2 can be followed.

A carrier oil (BERGABEST MCT Oil 60/40, Sternchemie GmbH & Co. KG) was combined with an industrial PL material (Lipogen PS20F, Lipogen Products (9000) Ltd.) having a sufficient moisture content to produce a PL-oil solution. The concentration of PLs to oil used was 65% w-w. A range of 10-80% w-w concentration was found to be amenable to the process with regard to solubility and stability.

The PL-oil solution was then combined with whole milk at a concentration of 2% w-w. A range of up to about 50% w-w concentration was found to be amenable to the process with regard to solubility and stability. The mixture was then stirred gently. Vigorous stirring at this stage can result in layer fractionation which produces cream. The PL-fortified milk was then homogenized. The PL-fortified milk was finally pasteurized to protect against spoilage.

A representative PL profile for the PL-fortified milk included: PS at 54%, PA at 18%, PC at 5%, PI at 18%, and PE at 5%. Percentages represent the weight fraction of the relevant PL component out of a total PL content based on PS, PA, PC, PI, and PE combined.

While the present invention has been described with respect to a limited number of embodiments, it will be appreciated that many variations, modifications, and other applications of the present invention may be made.

## Claims

1. A process for the preparation of phospholipid-enriched (PL-enriched) dairy products as nutraceuticals for the formulation of functional foods, the process comprising the steps of:
(a) combining a non-dairy-based PL-containing material with an oil component and water to form a paste;
(b) removing an excess amount of said water from said paste to form a PL-oil solution; and
(c) mixing said PL-oil solution with a dairy component, thereby obtaining a PL-enriched dairy product.

2. The process of claim 1, wherein said PL-enriched dairy product has the form of at least one nutraceutical type selected from the group consisting of: a medication, a medical food, a medical drink, a functional food, a functional drink, a nutritional supplement, a pharmaceutical supplement, and a dietary supplement.

3. The process of claim 1, wherein said PL-containing material includes at least one material selected from the group consisting of: a vegetal-derived lecithin, a non-vegetal-derived lecithin, a de-oiled lecithin, a native lecithin-oil solution, and an enzymatically-processed lecithin.

4. The process of claim 1, wherein said oil component includes at least one material selected from the group consisting of: a carrier oil and a native oil fraction from a lecithin production process.

5. The process of claim 1, wherein said step of combining is performed at a weight-to-weight (w-w) concentration of at least about 2% of said water to said PL-containing material.

6. The process of claim 1, wherein said step of removing is performed by at least one process selected from the group consisting of: a heating procedure, a vacuum-distillation procedure, and a solvent-based phase-separation procedure.

7. The process of claim 1, wherein said PL-oil solution has a weight-to-weight (w-w) concentration of at least about 0.01% of a residual amount of said water to said PL-containing material.

8. The process of claim 1, wherein said PL-oil solution has a weight-to-weight (w-w) concentration of about 10-80% of said PL-containing material to said oil component.

9. The process of claim 1, wherein said step of mixing includes at least one process selected from the group consisting of: a stirring procedure, a homogenizing procedure, and a pasteurizing procedure.

10. The process of claim 1, wherein said PL-enriched dairy product has a weight-to-weight (w-w) concentration of up to about 50% of said PL-oil solution to said dairy component.

11. The process of claim 1, wherein said PL-enriched dairy product is used in at least one product form selected from the group consisting of: a milk product, a chocolate product, a milk-ingredient supplemented product, an ice cream, a drink, a yoghurt, a processed cheese, a cottage cheese, a spread, a powdered dairy product, and a nutritional bar.

12. A nutraceutical and/or pharmaceutical composition produced according to the process of claim 1.

13. The nutraceutical composition of claim 12, wherein said PL-enriched dairy product has the form of at least one nutraceutical type selected from the group consisting of: a medication, a medical food, a medical drink, a functional food, a functional drink, a nutritional supplement, a pharmaceutical supplement, and a dietary supplement.

14. The nutraceutical composition of claim 12, wherein said PL-enriched dairy product is used in at least one product form selected from the group consisting of: a milk product, a chocolate product, a milk-ingredient supplemented product, an ice cream, a drink, a yoghurt, a processed cheese, a cottage cheese, a spread, a powdered dairy product, and a nutritional bar.
